(19) Europäisches Patentamt
European Patent Office
Office européen des brevets

(11) Publication number: **0 655 502 A1**

(12) **EUROPEAN PATENT APPLICATION**

(21) Application number: **94117445.0**

(22) Date of filing: **05.09.90**

(51) Int. Cl.[6]: **C12N 15/85**, C12N 5/10, C12N 15/53

This application was filed on 04 - 11 - 1994 as a divisional application to the application mentioned under INID code 60.

(30) Priority: **05.09.89 JP 228334/89**
**10.05.90 JP 118770/90**

(43) Date of publication of application:
**31.05.95 Bulletin 95/22**

(60) Publication number of the earlier application in accordance with Art.76 EPC: **0 421 139**

(84) Designated Contracting States:
**BE CH DE FR GB IT LI NL**

(71) Applicant: **NIPPON SUISAN KAISHA, LTD.**
**6-2, Ohtemachi 2-chome,**
**Chiyoda-ku**
**Tokyo (JP)**
Applicant: **NISSUI PHARMACEUTICAL CO., LTD.**
**11-1, Sugamo 2-chome**
**Toshima-ku,**
**Tokyo 170 (JP)**

(72) Inventor: **Yamashita, Shinya**
**Mezon Fuchu 301,**
**1-36-5, Miya-machi**
**Fuchu-shi,**
**Tokyo (JP)**
Inventor: **Hata, Junichiro**
**Jyunesu Takada A102,**
**8-24 Raiden-cho**
**Koga-shi,**
**Ibaragi-ken (JP)**
Inventor: **Kabeno, Shouko**
**3-16-305, 3-chome,**
**Kihei-cho**
**Kodaira-shi,**
**Toyko (JP)**
Inventor: **Matsumoto, Ken**
**2120, Shimoheimi,**
**Souwa-machi**
**Sashima-gun,**
**Ibaragi-ken (JP)**
Inventor: **Yagihashi, Satoru**
**Nissui Seiyaku Ryo,**
**474-1, Shimoheimi**
**Souwa-machi,**
**Sashima-gun,**
**Ibaragi-ken (JP)**
Inventor: **Kato, Hideo**
**5-16-7-202, Yutaka-cho**
**Kasukabe-shi,**
**Saitama-ken (JP)**

(74) Representative: **Wächtershäuser, Günter, Prof. Dr.**
**Patentanwalt,**
**Tal 29**
**D-80331 München (DE)**

(54) **Cho cells transformed with a recombinant plasmid expressing human secreting type thyroid peroxidase and preparation thereof.**

(57) There are given disclosures on a method of measuring an antimicrosormal antibody, a gene engineering process for preparing human thyroid peroxidase to be used for the measurement as well as an expression of the human thyroid peroxidase. The antimicrosormal antibody is measured by checking and investigating a reactivity of the human thyroid peroxidase produced by gene engineering techniques with a human serum. A natural membrane bound type human thyroid peroxidase or a secretion type human thyroid peroxidase is produced. The natural membrane bound type human peroxidase is produced on cell membrane of an cultivated animal cell or Escherichia coli transformed with an expression vector having therein recombinant DNAs encoding the human thyroid gene and dihydrofolate reductase gene. The secretion type human thyroid peroxidase is produced in a

EP 0 655 502 A1

supernatant of cultivated medium of animal cell transformed with an expression vector having therein recombinant DNAs encoding the human thyroid gene but with no membrane region thereof and dihydrofolate reductase gene.

The invention relates to a method of measuring an antimicrosormal antibody, a gene engineering process for the production of human thyroid peroxidase (hereinafter referred to as "hTPO") to be used for the measurement.

Most of all patients with a thyroid disease has in their serum autoantibodies capable of reacting with thyroglobulin, microsormal antigen and thyrotrophic hormone receptor. Among the autoantibodies, antimicrosormal antibody is quite useful for a diagnosis of autoimmune thyroid diseases, and it has been found that a value of the antibody shows a good interrelation with lymphatic infiltration in thyroid.

Further, it has been reported that the value increases in case of a patient with myasthenia grarsis, lupoid hepatitis, insulin dependent infantile diabetes and the like.

Therefore, a measurement of the antimicrosormal antibody can be utilized for a clinical diagnosis on said diseases.

Hitherto, a measurement of the antimicrosormal antibody has been carried out with various methods and, in general, following methods have been widely accepted, as testing methods.

a) Complement fixation test,
b) Fluorescent antibody test,
c) Passive hemagglutination test,
d) Precipitation test, and
e) E. I. A. (Enzyme Immuno Assay).

Since the nature of microsormal antigen has not sufficiently been elucidated, each of the above conventional methods uses, as an antigen, human thyroid tissue per se or a microsormal fraction obtained by somewhat purifying the tissue, so that it has substantial problems as in following matters.

1) Matter on differences due to origin of thyroid tissue;
2) Matters on humanity due to that the human tissue is employed as the reagent or raw material thereof and on availability of the tissue; and
3) Generation of non-specific reaction due to use of the human tissue or its fraction, which causes instability in sensitivity.

Recently, it has been so estimated that hTPO shall be nature of the antigen to autoantibodies produced in large amount in blood by the patient with thyroid disease. Further, a cloning on cDNA of hTPO has been carried out to make apparent its nucleotide sequence (Number of nucreotides : 3048, Molecular weight : 107000) and a primary structure of amino acids therefor (Shioko KIMURA et al, "Proc. Natl. Acad. Sci. USA", Vol. 84, pages 5555 - 5559, Aug. 1987, Biochemistry).

An object of the invention is to provide a method of measuring antimicrosormal antibodies, which is excellent in reaction specificity to show high sensitivity in measuring.

Another object of the invention is to provide a process for gene engineeringly preparing the hTPO as the nature of microsormal antigen, so that it make possible to supply the same as a reagent in stable and large amount and to finally solve the problems in the conventional methods, due to the humanistic ground and availability.

According to one of aspects of the invention, for attaining the objects, the invention utilizes the hTPO gene cloned by S. KIMURA et al disclosed in said literature, namely the cloned gene is inserted in an expression vector, an animal cell or Escherichia coli is transformed with the vector, and the transformant is cultivated to produce hTPO therein. The measurement of antimicrosormal antibodies can be carried out by using, as antigen, thus produced hTPO to check a reactivity thereof with serum sample.

In the first place, the insertion of expression vector into an animal cell shall be explained. The hTPO gene cloned by S. KIMURA et al has been inserted at EcoRI site of a plasmid (pUC8). In order to produce hTPO in animal cells, an operation is required to provide an initiation and termination of transcription to hTPO. Then , a plasmid (phTPO-1) with the hTPO gene, as disclosed by S. KIMURA et al in said literature, was digested by a restriction enzyme (EcoRI) to obtain a DNA fragment (about 3100bp) of the hTPO gene, While, a plasmid (pSV2-dhfr) known as one of vectors for an expression in animal cells was digested by restriction enzymes (HindIII and BglII) to obtain a DNA fragment (about 4000bp) with a SV40 promoter region and a replication initiation site in Escherichia coli.

In the next place, as a pre-treatment prior to re-construct a plasmid by ligating both of said DNA fragments, it is necessary to make each end of the fragments into a state connectable with each other. This pre-treatment can be carried out in a manner known per se. Namely it can be accomplished by adding a linker at a site cleaved by the restriction enzyme to give a common recognition site, or by treating with a DNA polymerase to make each end in blunt state.

The ligation of the both DNA fragments can be carried out with use of T4 ligase. Escherichia coli -(JM109 strain) was cultivated with use of reaction solution to cause a transformation thereof, screened to obtain ampicillin resistance colony, and then recovered a plasmid (pSV2-hTPO1) from the transformant. The plasmid is that dhfr (dihydrofolate reductase) gene in the plasmid (pSV2-dhfr) was recombined to hTPO gene, so that the dhfr gene usable as a drug resistance marker should be inserted therein to make complete the plasmid as that for expressing hTPO.

Therefore, the plasmid (pSV2-dhfr) was digested by restriction enzymes (PvuII and BglII) to obtain a DNA fragment (about 1100bp) with a SV40 promoter region and the dhfr gene. While, said re-constructed plasmid (pSV2-hTPO1) was cleaved with use of a restriction enzyme (EcoRI).

After pre-treatment in accordance with a manner known per se, both of said DNA fragments were ligated with use of T4 ligase. Escherichia coli (JM109 strain) was transformed with use of the solution for ligation reaction, screened to obtain an ampicillin resistance colony, and recovered a plasmid (pSV2-hTPO-dhfr) from the transformant.

A desired hTPO can be produced in stable and large amount, when the plasmid (pSV2-hTPO-dhfr) is inserted in a CHO cell (DXB11 strain) which is one of eukaryotic cells and widely employed as a host for production of substances with use of gene recombinant engineering techniques, and the resulting transformed cells were cultivated in a manner known per se.

In the following, another case of that an expression plasmid is inserted into Escherichia coli to produce hTPO shall be explained. In this case, also, it is started from the hTPO gene inserted plasmid (phTPO-1) disclosed by S. KIMURA et al, as in aforesaid case, the plasmid is digested and then re-constructed to prepare an expression plasmid. Namely, the plasmid (phTPO-1) is digested with use of restriction enzymes (EcoRI and XhoI) to obtain a DNA fragment (about 2900bp) with a translational region for hTPO gene and 3'-site untranslational region. While, a plasmid (pKK223-3) which is known as an expression vector in Escherichia coli is digested with use of a restriction enzyme (EcoRI) to obtain a DNA fragment.

Prior to a ligation operation for the fragments, following DNA linker (73bp for each strand) is prepared with use of a DNA synthesizer, so as to give a translation initiation codon of ATG which is necessary for an expression of DNA encoding a mature hTPO.

```
    EcoRI
            MetArgAlaLeuAlaValLeuSerValThrLeuValMetAlaCysThr
5' -AATTCATGAGAGCGCTCGCTGTGCTGTCTGTCACGCTGGTTATGGCCTGCACA
3' -    GTACTCTCGCGAGCGACACGACAGACAGTGCGACCAATACCGGACGTGT
                                                        XhoI
      GluAlaPhePheProPheIle
      GAAGCCTTCTTCCCCTTCATC
      CTTCGGAAGAAGGGGAAGTAGAGCT
```

In the presence of a phosphorylation reaction of the synthetic linker, the fragments as aforesaid are ligated with use of T4 ligase. Further, Escherichia coli (JM109 strain) was transformed with use of the ligating reaction solution, as in aforesaid case and an ampicillin resistance colony was obtained through a screening to recover a plasmid (pNKT-1) from the transformant, as in the manner given in the preceding case.

The desired hTPO can be produced by cultivating said transformed Escherichia coli, or by inserting said recombinant plasmid (pNKT-1) into Escherichia coli (JM109 strain) and cultivating the same.

For expressing in Escherichia coli a polypeptide including a portion for determining the antigen capable to react with autoantibodies of a patient, a part of the hTPO gene and not the entire there of may also be inserted into a plasmid to insert the same, in turn, into Escherichia coli. In this case, a plasmid (pKK233-2) may be employed as the expression vector and this plasmid vector shall be digested by a restriction enzyme (EcoRI) to make the same into a DNA fragment.

Further, if the plasmid (phTPO-1) with hTPO gene, disclosed by S. KIMURA et al in said literature, is digested with use of restriction enzymes (XhoI and AccI), a DNA fragment (about 2450bp) with a part of an open reading frame for the hTPO gene can be obtained, and a DNA fragment (about 970bp) with also a part of the open reading frame for the hTPO gene can be obtained, when the plasmid shall be digested with use of restriction enzymes of SmaI and AccI. Please note that these DNA fragments are obtained through a treatment by the restriction enzyme of AccI, so that those contain no membrane bound region of the hTPO gene.

These DNA fragment can be pre-treated in a manner known per se and ligated with the DNA fragment of said expression vector plasmid to re-construct into recombinant plasmids (pKT-XA and pKT-SA).

When the recombinant plasmid is inserted in Escherichia coli (JM109 strain) and the resulting transformant is cultivated, a substance including at least a part of the hTPO can be produced in the Escherichia coli.

The hTPO produced by the animal cell or Escherichia coli can be isolated and purified in a manner known per se. The resulting hTPO can be employed as the microsormal antigen for the measurement of antimicrosormal antibodies, which utilizes any of conventional methods.

Please note that the hTPO gene is one of membrane bound proteins, so that it expresses in a state bound to cell membrane, when the Escherichia coli or animal cell transformed with the hTPO gene is cultivated. It means that an amount of hTPO production is limited, that a mature cell or Escherichia coli must be broken to obtain hTPO in the form utilizable for the measurement of the antimicrosormal antibodies, to require troublesome purifying operations, even though it may be carried out in a conventional manner, and that there is a fear of that a region with relatively high hydrophobicity, to be considered as a portion binding to membrane may cause a bad influence to a host, when the entire gene of hTPO is inserted in the host of an animal cell or Escherichia coli for expression thereof, although such influence has not been confirmed.

According to another aspects of the invention, therefore, another basic object of the invention is to be made that hTPO can be produced not in the state bound to a membrane of an animal cell or Escherichia coli, but in the form of secretion, to make separation and purification easy, increase a productivity and allow an industrial scale production of the hTPO.

In connection with this aspect, the first concrete object of the invention is to provide an expression vector for the hTPO, suitable to secretional expression of hTPO by an animal cell.

The second concrete object of the invention is to provide a process for the preparation of such expression vector.

The third concrete object of the invention is to provide an animal cell transformed with said expression vector.

The fourth concrete object of the invention is to provide a process for obtaining hTPO with use of said transformed animal cell.

According to the invention, the basic object can be attained by cleaving off a membrane bound region in hTPO gene, giving a termination codon of TAA with use of a synthetic linker to modify the hTPO gene to that secretionally produces hTPO, and utilizing the modified gene.

The first concrete object of the invention can be attained by an expression vector suitable for secretional expression by animal cells, which comprises two promoter regions, a hTPO gene region with no membrane bound region, at downstream of one of the promoters, a dihydrofolate reductase (dhfr) gene region at downstream of the other promoter, and a terminator region between the hTPO gene region and dhfr region.

The second concrete object of the invention can be attained by a process comprising steps of cleaving a plasmid with cloned hTPO gene (phTPO1) with use of a restriction enzyme (AccI) to obtain a DNA fragment of about 5.7kb, ligating a synthetic linker to the DNA fragment, digesting the resulting DNA fragment with a restriction enzyme (EcoRI) to obtain a DNA fragment of about 2.8kb with a translational region for hTPO, reacting in the presence of T4 DNA ligase the DNA fragment with a plasmid (pUC19) digested by EcoRI, transforming Escherichia coli with use of the reaction solution, giving ampicillin resistance to the resulting transformant, recovering a plasmid with the hTPO gene (phTPO-EAL), cleaving the plasmid with a restriction enzyme (EcoRI) to obtain a DNA fragment of about 2.8kb, cleaving a plasmid (pSV2-dhfr) with use of restriction enzymes (HindIII and BglII to obtain a DNA fragment of about 4kb, end blunting each of the last two DNA fragments, causing to lost an enzymatic activity in each of the DNA fragments, causing a ligation reaction of the DNA fragments in the presence of T4 DNA ligase, transforming Escherichia coli with use of the reaction solution, giving ampicillin resistance to the resulting transformant, recovering a plasmid with the hTPO gene (pSV2-hTPO-EAL), cleaving a plasmid (pSV2-dhfr) with use of restriction enzymes (PvuIII and BglII and cleaving said re-constructed plasmid (pSV2-hTPO-EAL) with use or EcoRI, end blunting the resulting DNA fragments, ligating the DNA fragments with use of T4 DNA ligase to form a re-constructed plasmid, causing to lost enzymatic activity, and purifying the plasmid.

The synthetic linker to be used for the process may be of that having following nucreotide sequence.

5' -- AGACTAGTGAATTC -- 3'

5' -- TGATCACTTAAG -- 3'

The third concrete object of the invention can be attained by inserting the resulting hTPO expression vector in a animal cell to cause a transformation thereof. As the animal cell therefor, CHO cell which is one of eukaryotic cells may be used and the insertion can be carried out in a manner known per se.

The fourth concrete object of the invention can be attained by cultivating the animal cell transformed with the hTPO expression vector, and subjecting a supernatant in the cultivation medium to an affinity column treatment to adsorb the hTPO and then cause an elution thereof.

Fig. 1 is an illustration showing a manner to construct an expression vector which is suitable to express hTPO protein in an animal cell;

Fig. 2 is patterns showing results of that a molecular weight of hTPO expressed in transformed CHO cells was measured in accordance with western blotting method, in addition to molecular weight of controls, namely hTPO isolated from human thyroid tissue and product of non-transformed CHO cells;

Fig. 3 is an illustration showing a manner to construct an expression vector which is suitable to express in Escherichia coli;

Fig. 4 is an illustration showing manners to construct 2 expression vectors, in each of which a part of the hTPO gene is inserted;

Fig. 5 is a graph showing a result of computical analysis for amino acids encoding hTPO gene, searched from the view point of hydrophilicity - hydrophobicity of the amino acids;

Fig. 6 is an illustration showing a manner to construct an expression vector to express a secretion type hTPO;

Fig. 7 is patterns showing results of measurements, in accordance with a coomassy dyeing method, on molecular weight of a secretion type hTPO, a natural membrane bound type hTPO, a membrane bound type hTPO digested by trypcin as well as various molecular weight markers;

Fig. 8 is patterns showing results of measurements, in accordance with the western blotting, on molecular weight of the secretion type hTPO, natural membrane bound type hTPO, and membrane bound type hTPO digested by trypcin as well as various molecular weight markers; and

Fig. 9 is a graph showing results of search on reactivity of the secretion type hTPO and natural membrane bound type hTPO with serum samples of normal persons and serum samples of patients with autoimmune thyroid diseases.

The invention will now be further explained by Examples for preparing an hTPO expression vector, for inserting the vector into an animal cell or Escherichia coli, Example for measuring antimicrosormal antibodies, which uses the expressed hTPO, Test Examples and the like. Please note that Examples 1 - 6 as well as Test Examples 1 - 3 relate to a membrane bound type hTPO, but Examples 7 and 8 as well as Test Examples 4 and 5 to a secretion type hTPO.

Example 1

(Preparation of expression vector)

This Example shall be explained with reference to Fig. 1.

An hTPO gene cloned by S. KIMURA et al has been inserted at EcoRI recognition site of a plasmid (phTPO-1) with 5800bp.

To a solution of this plasmid (10μ g) in a mixture (50μ l) of 10mM Toris chloride buffer (pH 7.5), 7mM $MgCl_2$ and 60mM NaCl, a restriction enzyme (EcoRI, 10 units) was added to digest the plasmid at 37°C for 2 hours. A DNA fragment (about 2μ g) with the hTPO gene and of about 3100bp was obtained from the reaction solution by a DNA separation method of agarose gel electrophoresis.

While, to a solution of a plasmid (pSV2-dhfr, 10μ g) known as one of vectors expressing a products in animal cells, in a mixture (50μ l) of 10mM Toris chloride buffer (pH 7.5), 7mM $MgCl_2$ and 60mM NaCl, restriction enzymes (HindIII and BglII, each 10 units) were added to digest the plasmid at 37°C for 2 hours. A DNA fragment (about 3μ g) with SV40 promoter region, terminator region and replication origin in Escherichia coli and of about 4000bp was obtained from the reaction solution by the DNA separation method of agarose gel electrophoresis, as in the above.

To each of both DNA fragments (1μ g, respectively), DNA polymerase I (for large fragment) and a mixture of 6.6mM $MgCl_2$, 6.6mM Toris chloride buffer (pH 7.5), 50mM NaCl, 6.6mM mercaptoethanol and

500μ M dNTP were added to react at 23°C for 60 minutes. After the reaction, an extraction was carried out with phenol to inactivate the enzyme, and purify the DNA with use of the ethanol precipitation method to obtain each DNA fragment (about 0.5μ g) with blunt end.

To a mixture (50μ l) of 70mM Toris chloride buffer (pH 7.5), 10mM $MgCl_2$, 10mM DTT and 1mM ATP, each of said DNA fragments (0.5 μ g, respectively) was added to dissolve the same, and T4 ligase (5 units) was added therein to react at 16°C for 18 hours. With use of the reaction solution, Escherichia coli (JM109 strain) was transformed and an ampicillin resistance colony was obtained to recover a plasmid from the transformant (This recombinant plasmid had been named as "pSV2-hTPO1").

This plasmid corresponds to that the part of dhfr (dihydrofolate reductase) in the plasmid (pSV2-dhfr) was substituted with the hTPO gene. Since the dhfr gene is useful to give a drug resistance, an operation for inserting this gene into the plasmid (pSV2-hTPO1) shall be explained.

In the first place, the plasmid (pSV2-dhfr, 10μ g) was dissolved in a mixture (50μ l) of 10mM $MgCl_2$ and 60mM NaCl, and restriction enzymes (PvuII and BglII, each 10 units) were added to digest the plasmid at 37°C . The resulting reaction solution was subjected to agarose gel electrophoresis to obtain a DNA fragment (about 1100bp) with SV40 promoter region and dhfr gene region. While, aforesaid recombinant plasmid (pSV2-hTPO1, 1μ g) was dissolved in a mixture (50μ l) of 10mM Toris chloride buffer (pH 7.5), 7mM $MgCl_2$ and 60mM NaCl, and a restriction enzyme (EcoRI, 10 units) was added to digest the plasmid at 37°C for 2 hours, so that the plasmid (pSV2-hTPO1) was cleaved at its EcoRI recognition site.

To each of both DNA fragments (1μ g, respectively), DNA polymerase I (for large fragment, 1 unit) and a mixture (50μ l) of 6.6mM $MgCl_2$, 6.6mM Toris chloride buffer (pH 7.5), 50mM NaCl, 6.6mM mercaptoethanol and 500μ M dNTP were added to react at 23°C for 60 minutes. After the reaction, an extraction was carried out with phenol to inactivate the enzyme, and purify the DNA with use of ethanol precipitation method to obtain each DNA fragment (about 0.5μ g) with blunt end.

To a mixture (50μ l) of 70mM Toris chloride buffer (pH 7.5), 10mM $MgCl_2$, 10mM DTT and 1mM ATP, each of said DNA fragments (0.5 μ g, respectively) was added to dissolve the same, and T4 ligase (5 units) was added therein to react at 16°C for 18 hours. With use of the reaction solution, Escherichia coli (JM109 strain) was transformed and an ampicillin resistance colony was obtained to recover a plasmid as a desired expression vector, from the transformant (This recombinant plasmid had been named as "pSV2-hTPO-dhfr").

Reference Example

(Cultivation of CHO-dhfr cell)

As CHO-$dhfr^-$ which is one of eukaryotic cells, DXB11 strain established by Lawrence A. Chasin et al - ["Proc. Natl. Acad. Sci. U.S.A.", Vol. 77, No. 7, pages 4126 - 4220 (1980), hereinafter referred to mere as --CHO cell--] was employed.

A medium was prepared by adding $NaHCO_3$ (2.2g/l) in MEM α medium (marketed by GIBCO Co., No. 410-1900) to prepare a basic medium [hereinafter referred to as -- α (+) MEM medium--], and further adding thereto 5% dialyzed bovine fetus serum (hereinafter referred to as --D-FCS--). In this case, the D-FCS was prepared by dialyzing a bovine fetus serum for tissue culturing and marketed by Flow Co. (hereinafter referred to as --FCS--) to a phosphate buffer solution (--PBS-- having a composition of 8.0g/l NaCl, 0.2g/l KCl, 1.15g/l $Na_2HPO_4$ and 0.2g/l $KH_2PO_4$). The dialysis was carried out by adding FCS (200ml) to a dialyzing tube (size : 36/32), agitating the PBS (10 litres) kept at 4°C , and changing the PBS in entire amount over 3 times, after lapsed each time period of 10 hours. After completion of the dialysis, the dialized solution was passed through a filter (pore size : 0.2μ m) showing a low protein adsorption to remove possible bacteria. Then, the resulting filtrate was added to the medium.

The cultivation was carried out with use of a $CO_2$ incuvator, at 37°C , and under atmosphere of 5% $CO_2$ and saturation with water steam. A passage was carried out with an interval of 3 to 4 days and with a split ratio of 1 : 10 to 1 : 20, by washing with PBS and recover the cells with PBS, in which 0.05% trypcin (marketed by DIFCO Co.) and 0.02% EDTA were added.

Example 2

a) Insertion of vector and acquisition of transformants

The CHO cells cultivated and recovered as in Reference Example were washed with 5% D-FCS added α (+) MEM medium and a concentration thereof was adjusted to 5 x $10^7$ cells/ml with use of an insertion buffer [0.25M mannitol, 0.1mM $CaCl_2$, 0.1mM $MgCl_2$ and 0.2mM Tris-HCl (pH 7.2)]. While, the vector (pSV2-hTPO-dhfr) obtained in Example 1 was digested by a restriction enzyme (EcoRI), and a digested substance was recovered by ethanol precipitation method to dissolve the same into the insertion buffer to prepare 400μ g/ml solution.

The resulting vector solution and said cell suspension were mixed in equiamount and subjected to an electroporation. The electroporation was carried out with use of a cell fusing device (Type SSH-1) marketed by Shimazu Manufacturing Co., Ltd. and by setting pulse intensity of 3KV/cm, pulse width 50μ sec, pulse interval 1.0sec and number of pulse 2 times. The mixture (40μ l) was added in an insertion chamber (type C-12) and was left to stand, after the application of the pulse, under room temperature for about 10 minutes and then transferred, in equiamount, to 10 petri dishes (100mm in diameter) which accommodate the 5% D-FCS added α (+) MEM medium. After cultivating for about 48 hours, the medium was changed to α (-) MEM medium (marketed by GIBCO Co. No. 410-2000), in which 5% D-FCS was added, and the cultivation was continued, while changing the medium with a fresh one with an interval of 3 days. After 2 weeks from the initiation of cultivation, a colony of transformed cells was separated with use of a penicillin cup and recovered through trypcin treatment. The colony was subcultured with use of a multidish with 12 cell portions and then subjected to a screening, as stated below.

b) Screening of hTPO expression cell

The hTPO is a membrane enzyme of human thyroid follicular cells, so that a detection of hTPO expressed on CHO cells was carried out with use of a membrane immuno fluorescent assay (herein after referred to as --MIFA--).

In the first place, the transformed cells cultivated with the multidish were washed with PBS, recovered by a rubber policeman, suspended in PBS (100μ l), transferred to one of cell portions in a titer plate with 96 cell portions, centrifuged (1000rpm for 5 minutes) to remove supernatant therein, and suspended again in PBS (100μ l). After repeating 3 times of the centrifugal and suspending operations, a solution (100μ l) of rabbit anti-hTPO serum diluted 20 volumes with PBS was added and the resulting solution was left to stand for 1 hour at room temperature. After centrifugal washing 3 times with PBS, a solution (50μ l) of FITC labeled anti-rabbit IgG antibody (marketed by Medical and Biological Laboratory Co., Ltd.) diluted 20 volumes with PBS was added and the resulting solution was left to stand for 1 hour at room temperature. The centrifugal washing with PBS was repeated 3 times, and then the cells were suspended in 50μ l of PBS containing 50% glycerol, and a cell accommodating the cell suspension was mounted on a non-fluorescent slide glass to radiate an exciting lightbeam with use of a fluorescent microscope (marketed by Nikon Co., Ltd.).

A fluorescent cell is the desired one transformed and with an ability for producing hTPO.

Test Example 1

(Measurement of Molecular weight for produced hTPO)

The hTPO expressed cells confluently propagated in the petri dish [Item (b) in Example 2] were washed with PBS to add an ice-cooled CSS solution [10ml, containing 10mM Toris chloride buffer (pH 7.5), 150mM NaCl, 0.5% Toriton X-100 and 0.2mM phenylmethylsulphonylfluoride],left to stand for 15 minutes, collected with a rubber policeman, and centrifuged (1000rpm for 10 minutes) to obtain a supernatant as a cell solution.

To the cell solution (30μ l), a sample buffer [15μ l, containing 60mg/ml SDS, 150mM Toris chloride buffer (pH 6.8) 20% glycerol and 0.01% BPB] was added, subjected to an electrophoresis, and transcriped to a nitrocellurose membrane. The membrane was dipped in a blocking solution ("Blockace" marketed by Snow Brand Milk Products Co., Ltd.) for one overnight. The membrane was then dipped in a rabbit anti-hTPO serum diluted to 500 fold volumes with T-PBS (0.05% Tween added PBS) and mildly shaked. During the shaking, the solution of T-PBS was changed 3 times with fresh one with an interval of 15 minutes. The membrane was transferred to a solution of T-PBS containing $I^{125}$-labeled anti-rabbit IgG antibody (5μ Ci) to further shake mildly for 1 hour.

The resulting membrane was dried by air and an X-ray film was sensitized by the membrane at -70°C to detect a band identifying hTPO.

As controls, CHO cells not inserted the pSV2-hTPO-dhfr vector as well as a natural type hTPO isolated from a human thyroid tissue in accordance with the method as disclosed in J. Clin. Endocrinol. Metab., Vol. 63, page 570 (1986) were employed.

Results are shown in Fig. 2. As apparently from the Figure, it has been found that the molecular weight of hTPO produced by hTPO expression cell disclosed in Example 2 is about 107K daltons and same with that of the natural hTPO and that CHO cells inserted no pSV2-hTPO-dhfr vector does not express hTPO.

Test Example 2

(Reactivity of hTPO expressed by hTPO expressing cell with serum sample from patient with autoimmune thyroid disease)

Similar to MIFA described in Item (c) of Example 2 excepting that a serum of a patient with autoimmune thyroid diseases and diluted to 10 fold volumes with PBS was employed in lieu of rabbit anti-hTPO serum and that FITC labeled anti-human IgG antibody was used as a secondary antibody, a fluorescent intensity of cell surface was checked with use of a fluorescent microscope, as an index of an antigenous factor on hTPO produced by the hTPO expression cell. CHO cells inserted no pSV2-hTPO-dhfr were employed as a control.

Results are shown in following Table. As apparently seen therefrom, 24 serum samples among 28 samples show a reactivity to the expressed hTPO.

Table

| Samples | | Test | Control |
|---|---|---|---|
| 1 | C | + + + | + |
| 2 | G | + + | + |
| 3 | G | + + + | − |
| 4 | G | + + | − |
| 5 | G | ± | − |
| 6 | G | + + + | − |
| 7 | G | + + + | − |
| 8 | G | + + | ± |
| 9 | C | ± | ± |
| 10 | C | + | + |
| 11 | C | + | − |
| 12 | C | + | + |
| 13 | C | + + | − |
| 14 | C | + + + | ± |
| 15 | H | + + + | + |
| 16 | H | + + | − |
| 17 | G | + + | ± |
| 18 | G | + + + | − |
| 19 | G | + + | − |
| 20 | G | + | − |
| 21 | C | + + + | − |
| 22 | C | + + + | − |
| 23 | H | + + + | − |
| 24 | C | + + | − |
| 25 | H | + + | + |
| 26 | G | + + + | ± |
| 27 | H | + + + | ± |
| 28 | C | + + | ± |

In the Table,

| | | |
|---|---|---|
| G | : | Basedow's disease, |
| C | : | Chronic thyroiditis, |
| H | : | Hyperthyroidism, |
| − | : | Almost no fluorescence, |
| ± | : | Recognizable fluorescence, |
| + | : | Apparent florescence, and |
| + + | : | Strong fluorescence, and |
| + + + | : | Very strong fluorescence. |

Example 3

[Acquisition of cell strain with high hTPO expression ability, from methotrexate (MTX) resistance cell strains]

The hTPO expression cells (1 x $10^5$ cells) obtained by Example 2 was suspended in a medium (8ml) and transferred to a petri dish (10cm in diameter). The medium was prepared by adding 0.05$\mu$ M MTX in 5% FCS added $\alpha$ (-) MEM medium. The medium was changed to a fresh one with an interval of 3 days to continue the cultivation for about 2 weeks. A formed methotrexate resistance colony was recovered with a penicillin cup method to screen a cell strain showing high hTPO expression ability. In this case, some cell strains show a fluorescent intensity higher than that obtained by Example 2, so that such cell strains were recovered as those with high hTPO expression ability. Among 24 cell strains expressing hTPO, 4 strains show a high hTPO expression ability.

Example 4

(Preparation of expression vector)

This Example shall be explained with reference to Fig. 3.

In the first place, a hTPO gene inserted plasmid (phTPO-1, 5 $\mu$ g) disclosed by S. KIMURA et al was dissolved in a mixture (50 $\mu$ l) of 10mM Toris chloride buffer (pH 7.5), 7mM $MgCl_2$ and 100mM NaCl, and then restriction enzymes (EcoRI and XhoI, each 10 units) were added to the solution to digest the plasmid at 37°C for 2 hours. From the reaction solution, a DNA fragment (1$\mu$ g, about 2900bp) with hTPO translational region and 3'-site non-translational region was obtained by a DNA separation method of agarose gel electrophoresis.

While, to a solution of a plasmid (pKK223-3, 1$\mu$ g, marketed by Pharmacia Co.), known as one of vectors expressing a product in Escherichia coli, in a mixture (50$\mu$ l) of 10mM Toris chloride buffer (pH 7.5), 7mM $MgCl_2$ and 100mM NaCl, a restriction enzyme (EcoRI, 10 units) was added to digest the plasmid at 37°C for 2 hours. After inactivation of the enzyme by phenol extraction, ethanol precipitation method was carried out to purify and obtain a DNA fragment (pKK223-2, about 0.5$\mu$ g).

Following DNA linker (73bp for each strand) was prepared with use of a DNA synthesizer, so as to give a translational initiation codon of ATG which is necessary for expressing a DNA encoding a mature hTPO and as a pre-treatment for causing a ligation of said DNA fragments.

```
EcoRI
          MetArgAlaLeuAlaValLeuSerValThrLeuValMetAlaCysThr
5' -AATTCATGAGAGCGCTCGCTGTGCTGTCTGTCACGCTGGTTATGGCCTGCACA
3' -      GTACTCTCGCGAGCGACACGACAGACAGTGCGACCAATACCGGACGTGT
                                                        XhoI

   GluAlaPhePheProPheIle
   GAAGCCTTCTTCCCCTTCATC
   CTTCGGAAGAAGGGGAAGTAGAGCT
```

The synthesis was carried out by preparing each single-stranded DNA of 73 nucleotides, with use of the DNA synthesizer marketed by ABI Co., dissolving each strand (50pmol) in a mixture (50μ l) of 50mM Toris chloride buffer (pH 7.5), 10mM $MgCl_2$, 10mM DTT and 1mM ATP, adding T4 nucleotide kinase (5 units, marketed by Takara Shuzo Co., Ltd.) to phosphorylate at 37°C for 1 hour.

For a ligation of aforesaid two DNA fragments (0.5μ g, respectively) obtained by cleaving the concerned plasmid, each of the DNA fragments was taken by 0.5μ g, dissolved in a mixture (50 μ l) of 70mM Toris chloride buffer (pH 7.5), 10mM $MgCl_2$, 10mM DTT and 1mM ATP, and then the phosphorylation reaction solution of said synthetic linker (each 1μ l) was added thereto. To the resulting mixture, T4 ligase (5 units) was added to cause ligation reaction at 16°C for 18 hours. Escherichia coli (JM109 strain) was transformed with use of the resulting reaction solution and an ampicillin resistance colony was obtained through a screening to recover a plasmid (pNKT-1) from recovered transformant. A structure of this plasmid was checked through digestive experiments using various restriction enzymes.

Example 5

[Escherichia coli with plasmid (pNKT-1) and expression of hTPO by Escherichia coli]

The transformant described in Example 4 is the hTPO expression Escherichia coli. A desired hTPO expression Escherichia coli can also be prepared by inserting the recombinant plasmid (pNKT-1) obtained by Example 4 into Escherichia coli, in a manner known per se.

The Escherichia coli with the the plasmid (pNKT-1) was planted in LB medium (10g/l tryptone, 5g/l yeast extract and 5g/l NaCl), cultivated at 37°C to add isopropylthiogalactopyranoside (IPTG) in concentration of 50μ g/ml at a middle of logarithmic growth phase thereof and to continue the cultivation at 37°C , and then centrifuged to collect a solid. The solid was suspended in a sample buffer of laemli, subjected to SDS polyacryloamide gel electrophoresis, subjected to western blotting in accordance with the manner as described in "Proc. Natl. Acad. Sci. USA" Vol. 76, page 4350 (1979), and subjected to peroxidase dyeing in accordance with the manner as described by TABE in the literature "Saibou Kougaku" (translated as --Cell Engineerings--), Vol. 2, page 1061 (1983) to detect a band at position of molecular weight of 107K daltons. This means that the Escherichia coli with plasmid (pNKT-1) expresses hTPO.

Example 6

(Expression of polypeptide constituting a part of hTPO)

An experiment was carried out for the purpose of expressing in Escherichia coli a polypeptide containing an antigen determination site reacting with an autoantibody of patient, and for producing not the entire DNA for hTPO but a part thereof.

This Example shall be explained with reference to Fig. 4.

In the first place, a hTPO gene inserted plasmid (phTPO-1, 5 μ g) disclosed by S. KIMURA et al was dissolved in a mixture (50 μ l) of 10mM Toris chloride buffer (pH 7.5), 7mM $MgCl_2$ and 100mM NaCl, and then restriction enzymes (XhoI and AccI, each 10 units) were added to the solution to digest the plasmid at 37°C for 2 hours. From the reaction solution, a DNA fragment (about 1μ g, 2446bp) with a part of hTPO translational region was obtained by a DNA separation method of agarose gel electrophoresis.

Similar to the above using the plasmid (phTPO-1, 5μ g) but using restriction enzymes of SmaI and AccI (each 10 units), a DNA fragment (about 1μ g, 972bp) with a part of hTPO translational region was obtained from a reaction solution.

z Each of the both DNA fragments was dissolved in a mixture (50μ l) of 6.6mM $MgCl_2$, 6,6mM Toris chloride buffer (pH 7.5), 50mM NaCl, 6.6mM mercaptoethanol and 500μ M dNTP, and DNA polymerase I (for large fragment, 1 unit) was added to the solution to react at 23°C for 60 minutes. A phenol extraction was carried out on the reaction solution to inactivate the enzyme and the resulting extract was purified in accordance with ethanol precipitation method to obtain a DNA fragment (each 0.5μ g) with a blunt end.

While, to a solution of a plasmid (pKK223-2, 1μ g, marketed by Pharmacia Co.) in a mixture (50μ l) of 10mM Toris chloride buffer (pH 7.5), 7mM $MgCl_2$ and 100mM NaCl, a restriction enzyme (NcoI, 10 units) was added to digest the plasmid at 37°C for 2 hours. After inactivation of the enzyme by phenol extraction, ethanol precipitation method was carried out to purify and obtain a DNA fragment (pKK223-2, about 0.5μ g). The DNA fragment (about 1μ g) was dissolved in a mixture (50μ l) of 6.6mM $MgCl_2$, 6,6mM Toris chloride buffer (pH 7.5), 50mM NaCl, 6.6mM mercaptoethanol and 500μ M dNTP, and DNA polymerase I (1 unit) was added to the solution. A phenol extraction was carried out on the reaction solution to inactivate the enzyme and the resulting extract was purified in accordance with ethanol precipitation method to obtain a DNA fragment (about 0.5μ g) with a blunt end (this DNA fragment shall be referred to as "vector DNA fragment" for the sake of explanation).

The vector DNA is ligated with one of aforesaid 2 DNA fragments (with 2446bp and 972bp, respectively and with a blunt end) to re-construct 2 different expression vectors, but a manner of the ligation is same with each other. Namely, the vector DNA fragment and one of the DNA fragments, for instance that with 2446bp are dissolved in a mixture (50μ l) of 70mM Toris chloride buffer (pH 7.5), 10mM $MgCl_2$, 10mM DTT and 1mM ATP, and T4 ligase (5 units) was added thereto to react at 16°C for 18 hours to cause a ligation of the fragments. Escherichia coli (JM109 strain) was transformed with use of the resulting reaction solution and an ampicillin resistance colony was obtained through a screening. This transformant is the desired transformed Escherichia coli.

When a plasmid is recovered from this transformant, a desired recombinant expression plasmid (pKT-XA) can be obtained. While, if starting from the DNA fragment with 972bp, another desired plasmid (pKT-SA) can finally be obtained.

A structure of the plasmid (pKT-XA and pKT-SA) has been checked and confirmed through a digestion with use of various restriction enzymes.

Test Example 3

(Production of polypeptide containing a part of hTPO)

The transformant (Escherichia coli with plasmid (pKT-XA or pKT-SA) obtained by Example 6 was planted in LB medium, cultivated at 37°C to add IPTG in concentration of 50μ g/ml at a middle of logarithmic growth phase thereof and to continue the cultivation at 37°C , and then centrifuged to collect a solid. Similar to the manner as described in Example 5, the solid was suspended in a sample buffer of laemli, subjected to SDS polyacryloamide gel electrophoresis, subjected to western blotting using anti-hTPO polyclonal antibody, and subjected to peroxidase dyeing, so that bands at positions of molecular weight of 90K daltons and 49K daltons were detected for pKT-XA and pKT-SA, respectively. These bands can not be detected on a control of Escherichia coli with a plasmid of neither pKT-XA nor pKT-SA.

From the above, it has been confirmed that the Escherichia coli with the plasmid of pKT-XA or pKT-SA produces a part of polypeptide encoding hTPO.

Example 7

(Preparation of expression vector)

This Example shall be explained with reference to Figs. 5 and 6.

Fig. 5 is a graph showing a result of computical analysis for amino acids encoding the hTPO gene, searched from the view point of hydrophilicity - hydrophobicity of the amino acids. It is apparent from the graph that hTPO has a signal sequence at N-terminal and a membrane bound region at C-terminal, and that amino acids with relatively high hydrophobicity occupies therebetween.

In order to convert this natural membrane bound type hTPO gene into a secretion type hTPO gene, therefore, it is necessary to cleave off the membrane bound region at C-terminal. Operations therefor shall be explained below.

The hTPO gene (phTPO-1, 5μ g) cloned by S. KIMURA et al was dissolved in a mixture (50μ l) of 10mM Toris chloride buffer (pH 7.5), 7mM $MgCl_2$ and 100mM NaCl, and a restriction enzyme (AccI, 10 units) was added to digest the gene at 37°C for 2 hours. A DNA fragment (about 3μ g, about 5.7kb) with a translational region for the hTPO gene and a part of the vector region was obtained from the digestion reaction, solution by a separation method of agarose gel electrophoresis. The membrane bound region can be cleaved off from the translational region for hTPO gene through the digestion treatment.

Following two single-stranded DNA linkers were synthesized with use of a DNA synthesizer (marketed by ABI Co.), in order to give a termination codon and a restriction enzyme recognition site to the DNA fragment.

5' --- AGACTAGTGAATTC --- 3'

5' --- TGATCACTTAAG --- 3'

Each of the single-stranded DNA (50pmol) was dissolved in a mixture (50μ l) of 50mM Toris chloride buffer (pH 7.5), 10mM $MgCl_2$, 10mM DTT and 1mM ATP. To the resulting mixture, T4 polynucleotidekinase (5 units) was added to phosphorylate at 37°C for 1 hour. To said hTPO containing DNA fragment (1μ g) in a mixture (50μ l) of 70mM Toris chloride buffer (pH 7.5), 10mM $MgCl_2$, 10mM DTT and 1mM ATP, each of the reaction mixtures of phosphorylated DNA linkers was added by 1μ l. To the resulting mixture, T4 DNA ligase was added by 5 units to carry out a ligation at 16°C for 18 hours. To the reaction mixture, a restriction enzyme (EcoRI, 10 units) was added to carry out a digestion reaction at 37°C for 2 hours. A DNA separation method using an agarose gel was conducted to remove unreacted linker DNAs and vector region and to obtain a DNA fragment (0.5μ g, 2.8kb) with a hTPO translational region. This DNA fragment and another DNA fragment (0.1 μ g) obtained by digesting a plasmid (pUC19) with a restriction enzyme (EcoRI) were added in a mixture (50μ l) of 70mM Toris chloride buffer (pH 7.5), 10mM $MgCl_2$, 10mM DTT and 1mM ATP. To the resulting mixture, T4 DNA ligase was added by 5 units to carry out a ligation at 16°C for 18 hours. With use of the resulting reaction solution, Escherichia coli (JM109 strain) was transformed to obtain an ampicillin resistance colony. From the transformant, a plasmid (named as "phTPO-EAL") with a hTPO gene insert was recovered.

The plasmid (phTPO-EAL, 5μ g) constructed as above and with no membrane bound region was dissolved in a mixture (50μ l) of 10mM Toris chloride buffer (pH 7.5), 7mM $MgCl_2$ and 100mM NaCl. To the resulting mixture, a restriction enzyme (EcoRI, 10 units) was added to carry out a digestion reaction at 37°C for 2 hours. From the reaction solution, a DNA fragment (about 1μ g, about 2.8kb) with a translational region for hTPO gene but with no membrane bound region was obtained through a DNA separation method of agarose gel electrophoresis.

While, to a solution of a plasmid (pSV2-dhfr, 10μ g) known as one of vectors expressing a product in animal cells, in a mixture (50μ l) of 10mM Toris chloride buffer (pH 7.5), 7mM $MgCl_2$ and 60mM NaCl, restriction enzymes (HindIII and BglII, each 10 units) were added to digest the plasmid at 37°C for 2 hours. A DNA fragment (about 3μ g, about 4kb) with SV40 promoter region, terminator region and replication origin in Escherichia coli was obtained from the reaction solution by the DNA separation method of agarose gel electrophoresis.

To each of this DNA fragment and aforesaid hTPO gene containing DNA fragment (1μ g, respectively), DNA polymerase I (for large fragment) and a mixture (50μ l) of 6.6mM Toris chloride buffer (pH 7.5), 6.6mM $MgCl_2$, 50mM NaCl, 6.6mM mercaptoethanol and 500μ M dNTP were added to react at 23°C for 60 minutes. After the reaction, an extraction was carried out with use of phenol to inactivate the enzyme and an extract was purified through ethanol precipitation method to obtain each DNA fragment (about 0.5μ g) with blunt end.

To a mixture (50μ l) of 70mM Toris chloride buffer (pH 7.5), 10mM $MgCl_2$, 10mM DTT and 1mM ATP, each of said DNA fragments (0.5 μ g, respectively) was added to dissolve the same, and T4 ligase (5 units) was added therein to react at 16°C for 18 hours. With use of the reaction solution, Escherichia coli (JM109 strain) was transformed and an ampicillin resistance colony was obtained to recover a plasmid from the transformant (This recombinant plasmid had been named as "pSV2-hTPO-EAL".

This plasmid corresponds to that the part of dhfr (dihydrofolate reductase) in the plasmid (pSV2-dhfr) was substituted with the hTPO gene. Since the dhfr gene to be cleaved off through said treatment is useful to give a drug resistance, an operation for inserting this gene into the plasmid (pSV2-hTPO-EAL) shall be explained.

In the first place, the plasmid (pSV2-dhfr, 5μ g) was dissolved in a mixture (50μ l) of 10mM Toris chloride buffer (pH 7.5), 7mM $MgCl_2$ and 100mM NaCl, and restriction enzymes (PvuII and BglII, each 10 units) were added to digest the plasmid at 37°C for 2 hours. The resulting reaction solution was subjected to agarose gel electrophoresis to obtain a DNA fragment (about 2μ g, about 1.1kb) with SV40 promoter region and dhfr gene region. While, aforesaid recombinant plasmid (pSV2-hTPO-EAL, 1μ g) was dissolved in a mixture (50μ l) of 10mM Toris chloride buffer (pH 7.5), 7mM $MgCl_2$ and 100mM NaCl, and a restriction enzyme (EcoRI, 10 units) was added to digest the plasmid at 37°C for 2 hours, so that the plasmid (pSV2-hTPO-EAL) was cleaved at its EcoRI recognition site.

To each of both DNA fragments (1μ g, respectively), DNA polymerase I (for large fragment, 1 unit) and a mixture (50μ l) of 6.6mM $MgCl_2$, 6.6mM Toris chloride buffer (pH 7.5), 50mM NaCl, 6.6mM mercaptoethanol and 500μ M dNTP were added to react at 23°C for 60 minutes. After the reaction, an extraction was carried out with phenol to inactivate the enzyme, and an extract was purified through ethanol precipitation method to obtain each DNA fragment (about 0.5μ g) with blunt end.

To a mixture (50μ l) of 70mM Toris chloride buffer (pH 7.5), 1mM $MgCl_2$, 10mM DTT and 1mM ATP, each of said DNA fragments (0.5 μ g, respectively) was added to dissolve the same, and T4 ligase (5 units) was added therein to react at 16°C for 18 hours. With use of the reaction solution, Escherichia coli (JM109 strain) was transformed and an ampicillin resistance colony was obtained to recover a plasmid as a desired expression vector, from the transformant (This recombinant plasmid had been named as "pSV2-hTPO-EAL-dhfr").

Example 8

a) Cultivation of CHO-dhfr cell

Same with that as described in Reference Example.

b) Insertion of expression vector and acquisition of transformants

Same with that as described in Item a) in Example 2, accepting that the vector (pSV2-hTPO-EAL-dhfr) obtained by Example 7 was inserted in CHO cell, in lieu of the vector (pSV2-hTPO-dhfr) for Example 2.

c) Screening of secretion type hTPO expression cell

The natural hTPO is one of membrane bound proteins. When CHO cells transformed with the hTPO gene are cultivated, therefore, hTPO to be produced will appears on a cell membrane.

However, the recombinant CHO cell obtained in the preceding Item b) has been transformed by the gene, wherein the membrane bound region was removed from the natural type hTPO gene, as apparently seen from the description given in Example 7, so that the desired product of hTPO protein will be secreted by the transformant into a cultivation medium.

Therefore, an amount of hTPO secreted in a supernatant of the medium was measured by an enzyme immuno assay. Namely, the transformant was cultivated in a multidish with 12 cell portions to obtain a supernatant in the cultivation medium. The supernatant was transferred into one of 96 cell portions of a titer plate to leave to stand for 2 hours at 37°C . After removal of a supernatant, a residue was washed 3 times with PBS, and a blocking solution (200μ l, "Blockace" marketed by Snow Brand Milk Products Co., Ltd.) diluted to 4 fold volumes was added to leave to stand for 1 hour at 37°C . After washed 3 times with PBS, rabbit anti-hTPO serum (100μ l) diluted to 1000 fold volume with PBS was added to leave to stand for 1 hour at 37°C . Then washed 3 times with PBS. an anti-rabbit IgG solution (100μ , marketed by TAGO Co.) labeled by alkaline phosphatase and diluted to 500 fold volume was added to leave to stand for 1 hour at 37°C . After further washed 3 times with PBS, s substrate solution (100μ l and having a composition of 1.53g/l $Na_2CO_3$, 0.84g/l $NaHCO_3$, 0.98g/l phenylphosphate• 2Na and 0.4g/l 4-aminoampicillin) was added to leave to stand for 30 minutes at room temperature. Thereafter, a color reagent (8g/l $NaIO_4$) was added to measure an absorbance of the resulting solution at 490nm. A desired cell having hTPO expression ability shows a high value in absorbance.

d) Acquisition of cell with high hTPO expression ability

A cell showed highest value in absorbance among cell strains obtained through the experiments given in said Item c) was named as "STF-1". The STF-1 cell (1 x $10^5$ cells) were suspended in a medium (8ml) and transferred to a petri dish of 100mm in diameter. The medium in this case was prepared by adding 0.02μ M MTX in 5% FCS added α (-) MEM medium. The medium was changed to a fresh one with an interval of about 3 days to continue the cultivation for about 2 weeks. A formed MTX resistance colony was recovered with penicillin cup method to screen a cell with high hTPO expression ability, with use of the enzyme immuno assay, as described in said Item c).

A cell strain with more higher hTPO expression ability can be obtained by repeating a screening as referred to, excepting that the concentration of MTX is increased.

Finally, 0.1% MTX resistance cell was obtained and named as "STT-1".

e) Scale-up cultivation of STT-1 cell strain

The STT-1 cells obtained in said Item d) were suspended in a culture medium (30ml) by 4 x $10^5$ cells and transferred to two petri dishes of 150mm in diameter. The medium in this case was prepared by adding 0.1μ M MTX in 5% FCS added α (-) MEM medium. The medium was changed to a fresh one with an interval of about 3 days to continue the cultivation for about 9 days, so that a supernatant of the cultivation medium was obtained by 150ml in total. An amount of hTPO in the supernatant was measured by an enzyme immuno assay to find out that it contains hTPO of 40ng. For purifying the hTPO, the supernatant was adsorbed to and eluted from an anti-hTPO monoclonal antibody binding affinity column developed by KOTANI et al ["J. Clin. Endocino. Metab.", Vol. 63, page 570 (1986)] to check an amount of hTPO by the enzyme immuno assay, It showed that the elute contains hTPO by 5μ g/ml.

Test Example 4

(Measurement of molecular weight on secretion type hTPO)

A solution containing hTPO (5μ g/ml), which was obtained and purified through affinity column chromatography as described in Example 8 was freeze-dried and dissolved again in water to obtain a solution (50μ l) containing hTPO in a concentration of 100ng/μ l. To this hTPO solution, a sample buffer solution [15μ l with a composition of 60mg/ml SDS, 150mM Toris chloride buffer (pH 6.8), 20% glycerol and 0.01% BPB] was added and the resulting mixture was subjected to 8% polyacryloamide gel electrophoresis. A transcription to a nitrocellulose membrane was carried out and the membrane was dipped in a blocking solution [same with that referred in Item c) of Example 8)] for one overnight. The membrane was then dipped in a rabbit anti-hTPO serum diluted in 500 volumes with T-PBS (0.05% Tween added PBS) and mildly shaked for 1 hour (In this case, T-TBS was changed 3 times with an interval of 15 minutes). The membrane was dipped in biotinized anti-rabbit IgG solution diluted in 500 volumes to mildly shake the same for 1 hour. After washed 3 times with PBS, the membrane was reacted with ABS reagent for 30 minutes, and then washed again 3 times with PBS. The resulting membrane was dipped in a substrate solution [5mg dianisidine, 30μ l $H_2O_2$, and 1ml/100ml 1M Toris chloride buffer (pH 7.5)] to leave to stand for 15 minutes at room temperature to detect a band. Results are shown in Fig. 8. In the above experiments according to western blotting, an ABC kit (marketed by Vector Lab. Co.) was employed.

Results of another experiments, wherein aforesaid hTPO solution was subjected to electrophoresis, as in the above experiment, and then treated in accordance with a coomassy dyeing method, are shown in Fig. 7.

In both experiments, a natural membrane bound type hTPO isolated from human thyroid tissue in accordance with the method as disclosed in "J. Clin. Endocinol. Metab.", Vol. 63, page 570 (1986) had been employed as a control.

As apparently seen from Figs. 7 and 8, the natural type hTPO protein shows a molecular weight of 107K daltons, but the molecular weight of secretion type hTPO reduces therefrom by that for the membrane bound region to show 101K daltons.

Test Example 5

(Reactivity of membrane bound type hTPO and secretion type hTPO with serum of patient with autoimmune thyroid diseases)

A reactivity of the secretion type hTPO and natural membrane bound type hTPO with a serum sample from patients with autoimmune thyroid diseases was checked in accordance with the enzyme immuno assay similar to that as referred to in Item c) of Example 8, excepting that serum of patients with autoimmune thyroid diseases diluted in 10 volumes with PBS was employed in lieu of the rabbit anti-hTPO serum, biotinized human anti-IgG solution was employed in lieu of the biotinized rabbit anti-IgG solution, and that an absorbance was measured at 492nm to make an index showing an antigenous factor of hTPO, in lieu of the measurement wave length of 490nm. Results are shown in Fig. 9. As apparently seen from the Figure, normal serum sample shows almost no reactivity with each of the hTPOs but, on the contrary thereto, serum sample from patient with autoimmune thyroid diseases shows definite reactivity with each of the secretion type hTPO and natural membrane bound type hTPO.

The deposition numbers on the CHO-cell-lines have been given, as follows:

a) CHO cell-line transformed with a vector plasmid to express a membrane bound type hTPO:
Deposition No. FERM BP-3076
Deposition date: August 24, 1990

b) CHO cell-line transformed with a vector plasmid to express a secretion type hTPO:
Deposition No. FERM BP-3077
Deposition date: August 24, 1990

```
SEQ ID NO          : 1
SEQUENCE TYPE      : Nucleotide with corresponding protein
SEQUENCE LENGTH    : 3048 base pairs

STRANDEDNESS       : double
TOPOLOGY           : linear
MOLECULE TYPE      : cDNA to mRNA

ORIGINAL SOURCE
ORGANISM           : human

IMMEDIATE EXPERIMENTAL SOURCE
NAME OF CELL LINE : human thyroid from Graves disease

FEATURES           :

PRPERTIES          : human thyroid peroxidase gene
```

```
CATTTCAGAA GAGTTACAGC CGTGAAAATT ACTCAGCAGT GCAGTTGGCT GAGAAGAGGA      60

AAAAAGGTCA GA ATG AGA GCG CTC GCT GTG CTG TCT GTC ACG CTG GTT ATG     111
              Met Arg Ala Leu Ala Val Leu Ser Val Thr Leu Val Met

GCC TGC ACA GAA GCC TTC TTC CCC TTC ATC TCG AGA GGG AAA GAA CTC      159
Ala Cys Thr Glu Ala Phe Phe Pro Phe Ile Ser Arg Gly Lys Glu Leu

CTT TGG GGA AAG CCT GAG GAG TCT CGT GTC TCT AGC GTC TTG GAG GAA      207
Leu Trp Gly Lys Pro Glu Glu Ser Arg Val Ser Ser Val Leu Glu Glu

AGC AAG CGC CTG GTG GAC ACC GCC ATG TAC GCC ACG ATG CAG AGA AAC      255
Ser Lys Arg Leu Val Asp Thr Ala Met Tyr Ala Thr Met Gln Arg Asn

CTC AAG AAA AGA GGA ATC CTT TCT CCA GCT CAG CTT CTG TCT TTT TCC      303
Leu Lys Lys Arg Gly Ile Leu Ser Pro Ala Gln Leu Leu Ser Phe Ser

AAA CTT CCT GAG CCA ACA AGC GGA GTG ATT GCC CGA GCA GCA GAG ATA      351
Lys Leu Pro Glu Pro Thr Ser Gly Val Ile Ala Arg Ala Ala Glu Ile

ATG GAA ACA TCA ATA CAA GCG ATG AAA AGA AAA GTC AAC CTG AAA ACT      399
Met Glu Thr Ser Ile Gln Ala Met Lys Arg Lys Val Asn Leu Lys Thr

CAA CAA TCA CAG CAT CCA ACG GAT GCT TTA TCA GAA GAT CTG CTG AGC      447
Gln Gln Ser Gln His Pro Thr Asp Ala Leu Ser Glu Asp Leu Leu Ser

ATC ATT GCA AAC ATG TCT GGA TGT CTC CCT TAC ATG CTG CCC CCA AAA      495
Ile Ile Ala Asn Met Ser Gly Cys Leu Pro Tyr Met Leu Pro Pro Lys

TGC CCA AAC ACT TGC CTG GCG AAC AAA TAC AGG CCC ATC ACA GGA GCT      543
Cys Pro Asn Thr Cys Leu Ala Asn Lys Tyr Arg Pro Ile Thr Gly Ala

TGC AAC AAC AGA GAC CAC CCC AGA TGG GGC GCC TCC AAC ACG GCC CTG      591
Cys Asn Asn Arg Asp His Pro Arg Trp Gly Ala Ser Asn Thr Ala Leu

GCA CGA TGG CTC CCT CCA GTC TAT GAG GAC GGC TTC AGT CAG CCC CGA      639
Ala Arg Trp Leu Pro Pro Val Tyr Glu Asp Gly Phe Ser Gln Pro Arg

GGC TGG AAC CCC GGC TTC TTG TAC AAC GGG TTC CCA CTG CCC CCG GTC      687
Gly Trp Asn Pro Gly Phe Leu Tyr Asn Gly Phe Pro Leu Pro Pro Val

CGG GAG GTG ACA AGA CAT GTC ATT CAA GTT TCA AAT GAG GTT GTC ACA      735
Arg Glu Val Thr Arg His Val Ile Gln Val Ser Asn Glu Val Val Thr

GAT GAT GAC CGC TAT TCT GAC CTC CTG ATG GCA TGG GGA CAA TAC ATC      783
Asp Asp Asp Arg Tyr Ser Asp Leu Leu Met Ala Trp Gly Gln Tyr Ile

GAC CAC GAC ATC GCG TTC ACA CCA CAG AGC ACC AGC AAA GCT GCC TTC      831
Asp His Asp Ile Ala Phe Thr Pro Gln Ser Thr Ser Lys Ala Ala Phe

GGG GGA GGG GCT GAC TGC CAG ATG ACT TGT GAG AAC CAA AAC CCA TGT      879
Gly Gly Gly Ala Asp Cys Gln Met Thr Cys Glu Asn Gln Asn Pro Cys

TTT CCC ATA CAA CTC CCG GAG GAG GCC CGG CCG GCC GCG GGC ACC GCC      927
Phe Pro Ile Gln Leu Pro Glu Glu Ala Arg Pro Ala Ala Gly Thr Ala
```

```
TGT CTG CCC TTC TAC CGC TCT TCG GCC GCC TGC GGC ACC GGG GAC CAA      975
Cys Leu Pro Phe Tyr Arg Ser Ser Ala Ala Cys Gly Thr Gly Asp Gln

GGC GCG CTC TTT GGG AAC CTG TCC ACG GCC AAC CCG CGG CAG CAG ATG     1023
Gly Ala Leu Phe Gly Asn Leu Ser Thr Ala Asn Pro Arg Gln Gln Met

AAC GGG TTG ACC TCG TTC CTG GAC GCG TCC ACC GTG TAT GGC AGC TCC     1071
Asn Gly Leu Thr Ser Phe Leu Asp Ala Ser Thr Val Tyr Gly Ser Ser

CCG GCC CTA GAG AGG CAG CTG CGG AAC TGG ACC AGT GCC GAA GGG CTG     1119
Pro Ala Leu Glu Arg Gln Leu Arg Asn Trp Thr Ser Ala Glu Gly Leu

CTC CGC GTC CAC GCG CGC CTC CGG GAC TCC GGC CGC GCC TAC CTG CCC     1167
Leu Arg Val His Ala Arg Leu Arg Asp Ser Gly Arg Ala Tyr Leu Pro

TTC GTG CCG CCA CGG CGG CCT GCG GCC TGT GCG CCC GAG CCC GGC ATC     1215
Phe Val Pro Pro Arg Arg Pro Ala Ala Cys Ala Pro Glu Pro Gly Ile

CCC GGA GAG ACC CGC GGG CCC TGC TTC CTG GCC GGA GAC GGC CGC GCC     1263
Pro Gly Glu Thr Arg Gly Pro Cys Phe Leu Ala Gly Asp Gly Arg Ala

AGC GAG GTC CCC TCC CTG ACG GCA CTG CAC ACG CTG TGG CTG CGC GAG     1311
Ser Glu Val Pro Ser Leu Thr Ala Leu His Thr Leu Trp Leu Arg Glu

CAC AAC CGC CTG GCC GCG GCG CTC AAG GCC CTC AAT GCG CAC TGG AGC     1359
His Asn Arg Leu Ala Ala Ala Leu Lys Ala Leu Asn Ala His Trp Ser

GCG GAC GCC GTG TAC CAG GAG GCG CGC AAG GTC GTG GGC GCT CTG CAC     1407
Ala Asp Ala Val Tyr Gln Glu Ala Arg Lys Val Val Gly Ala Leu His

CAG ATC ATC ACC CTG AGG GAT TAC ATC CCC AGG ATC CTG GGA CCC GAG     1455
Gln Ile Ile Thr Leu Arg Asp Tyr Ile Pro Arg Ile Leu Gly Pro Glu

GCC TTC CAG CAG TAC GTG GGT CCC TAT GAA GGC TAT GAC TCC ACC GCC     1503
Ala Phe Gln Gln Tyr Val Gly Pro Tyr Glu Gly Tyr Asp Ser Thr Ala

AAC CCC ACT GTG TCC AAC GTG TTC TCC ACA GCC GCC TTC CGC TTC GGC     1551
Asn Pro Thr Val Ser Asn Val Phe Ser Thr Ala Ala Phe Arg Phe Gly

CAT GCC ACG ATC CAC CCG CTG GTG AGG AGG CTG GAC GCC AGC TTC CAG     1599
His Ala Thr Ile His Pro Leu Val Arg Arg Leu Asp Ala Ser Phe Gln

GAG CAC CCC GAC CTG CCC GGG CTG TGG CTG CAC CAG GCT TTC TTC AGC     1647
Glu His Pro Asp Leu Pro Gly Leu Trp Leu His Gln Ala Phe Phe Ser

CCA TGG ACA TTA CTC CGT GGA GGT GGT TTG GAC CCA CTA ATA CGA GGC     1695
Pro Trp Thr Leu Leu Arg Gly Gly Gly Leu Asp Pro Leu Ile Arg Gly

CTT CTT GCA AGA CCA GCC AAA CTG CAG GTG CAG GAT CAG CTG ATG AAC     1743
Leu Leu Ala Arg Pro Ala Lys Leu Gln Val Gln Asp Gln Leu Met Asn

GAG GAG CTG ACG GAA AGG CTC TTT GTG CTG TCC AAT TCC AGC ACC TTG     1791
Glu Glu Leu Thr Glu Arg Leu Phe Val Leu Ser Asn Ser Ser Thr Leu
```

GAT CTG GCG TCC ATC AAC CTG CAG AGG GGC CGG GAC CAC GGG CTG CCA 1839
Asp Leu Ala Ser Ile Asn Leu Gln Arg Gly Arg Asp His Gly Leu Pro

GGT TAC AAT GAG TGG AGG GAG TTC TGC GGC CTG CCT CGC CTG GAG ACC 1887
Gly Tyr Asn Glu Trp Arg Glu Phe Cys Gly Leu Pro Arg Leu Glu Thr

CCC GCT GAC CTG AGC ACA GCC ATC GCC AGC AGG AGC GTG GCC GAC AAG 1935
Pro Ala Asp Leu Ser Thr Ala Ile Ala Ser Arg Ser Val Ala Asp Lys

ATC CTG GAC TTG TAC AAG CAT CCT GAC AAC ATC GAT GTC TGG CTG GGA 1983
Ile Leu Asp Leu Tyr Lys His Pro Asp Asn Ile Asp Val Trp Leu Gly

GGC TTA GCT GAA AAC TTC CTC CCC AGG GCT CGG ACA GGG CCC CTG TTT 2031
Gly Leu Ala Glu Asn Phe Leu Pro Arg Ala Arg Thr Gly Pro Leu Phe

GCC TGT CTC ATT GGG AAG CAG ATG AAG GCT CTG CGG GAT GGT GAC TGG 2079
Ala Cys Leu Ile Gly Lys Gln Met Lys Ala Leu Arg Asp Gly Asp Trp

TTT TGG TGG GAG AAC AGC CAC GTC TTC ACG GAT GCA CAG AGG CGT GAG 2127
Phe Trp Trp Glu Asn Ser His Val Phe Thr Asp Ala Gln Arg Arg Glu

CTG GAG AAG CAC TCC CTG TCT CGG GTC ATC TGT GAC AAC ACT GGC CTC 2175
Leu Glu Lys His Ser Leu Ser Arg Val Ile Cys Asp Asn Thr Gly Leu

ACC AGG GTG CCC ATG GAT GCC TTC CAA GTC GGC AAA TTC CCT GAA GAC 2223
Thr Arg Val Pro Met Asp Ala Phe Gln Val Gly Lys Phe Pro Glu Asp

TTT GAG TCT TGT GAC AGC ATC CCT GGC ATG AAC CTG GAG GCC TGG AGG 2271
Phe Glu Ser Cys Asp Ser Ile Pro Gly Met Asn Leu Glu Ala Trp Arg

GAA ACC TTT CCT CAA GAC GAC AAG TGT GGC TTC CCA GAG AGC GTG GAG 2319
Glu Thr Phe Pro Gln Asp Asp Lys Cys Gly Phe Pro Glu Ser Val Glu

AAT GGG GAC TTT GTG CAC TGT GAG GAG TCT GGG AGG CGC GTG CTG GTG 2367
Asn Gly Asp Phe Val His Cys Glu Glu Ser Gly Arg Arg Val Leu Val

TAT TCC TGC CGG CAC GGG TAT GAG CTC CAA GGC CGG GAG CAC CTC ACT 2415
Tyr Ser Cys Arg His Gly Tyr Glu Leu Gln Gly Arg Glu Gln Leu Thr

TGC ACC CAG GAA GGA TGG GAT TTC CAG CCT CCC CTC TGC AAA GAT GTG 2463
Cys Thr Gln Glu Gly Trp Asp Phe Gln Pro Pro Leu Cys Lys Asp Val

AAC GAG TGT GCA GAC GGT GCC CAC CCC CCC TGC CAC GCC TCT GCG AGG 2511
Asn Glu Cys Ala Asp Gly Ala His Pro Pro Cys His Ala Ser Ala Arg

TGC AGA AAC ACC AAA GGC GGC TTC CAG TGT CTC TGC GCG GAC CCC TAC 2559
Cys Arg Asn Thr Lys Gly Gly Phe Gln Cys Leu Cys Ala Asp Pro Tyr

GAG TTA GGA GAC GAT GGG AGA ACC TGC GTA GAC TCC GGG AGG CTC CCT 2607
Glu Leu Gly Asp Asp Gly Arg Thr Cys Val Asp Ser Gly Arg Leu Pro

CGG GCG ACT TGG ATC TCC ATG TCG CTG GCT GCT CTG CTG ATC GGA GGC 2655
Arg Ala Thr Trp Ile Ser Met Ser Leu Ala Ala Leu Leu Ile Gly Gly

TTC GCA GGT CTC ACC TCG ACG GTG ATT TGC AGG TGG ACA CGC ACT GGC 2703
Phe Ala Gly Leu Thr Ser Thr Val Ile Cys Arg Trp Thr Arg Thr Gly

```
ACT AAA TCC ACA CTG CCC ATC TCG GAG ACA GGC GGA GGA ACT CCC GAG      2751
Thr Lys Ser Thr Leu Pro Ile Ser Glu Thr Gly Gly Gly Thr Pro Glu

CTG AGA TGC GGA AAG CAC CAG GCC GTA GGG ACC TCA CCG CAG CGG GCC      2799
Leu Arg Cys Gly Lys His Gln Ala Val Gly Thr Ser Pro Gln Arg Ala

GCA GCT CAG GAC TCG GAG CAG GAG AGT GCT GGG ATG GAA GGC CGG GAT      2847
Ala Ala Gln Asp Ser Glu Gln Glu Ser Ala Gly Met Glu Gly Arg Asp

ACT CAC AGG CTG CCG AGA GCC CTC TGA GGGCAAAGTG GCAGGACACT GCAGAACAGC 2904
Thr His Arg Leu Pro Arg Ala Leu

TTCATGTTCC CAAAATCACC GTACGACTCT TTTCCAAACA CAGGCAAATC GGAAATCAGC    2964

AGGACGACTG TTTTCCCAAC ACGGGTAAAT CTAGTACCAT GTCGTAGTTA CTCTCAGGCA    3024

TGGATGAATA AATGTTATAG CTGCAN                                         3048
```

## Claims

1. A plasmid suitable for an expression of a secreting protein part of human thyroid peroxidase with a CHO cell, which comprises:
   (a) a DNA sequence coding for a first protein part of human thyroid peroxidase region retaining activity of antigen and being substantially free of a sequence coding for a membrane binding region;
   (b) a first promoter sequence controlling an expression of the DNA sequence coding for the first protein ;
   (c) a DNA sequence coding for a second protein dihydrofolate reductase;
   (d) a second promoter sequence controlling an expression of the DNA sequence coding for the second protein, said DNA sequence coding for the first protein and said DNA sequence coding for the second protein being situated between said first promoter sequence and said second promoter sequence, and said both promoter sequences facing to the opposite direction each other; and
   (e) the only one terminator sequence common to said DNA sequence coding for the first protein and said DNA sequence coding for the second protein, being situated between said both sequences coding for each protein.

2. The plasmid according to Claim 1, wherein said first and second promoter sequences are each derived from an SV40 promoter sequence, and said terminator sequence is derived from an SV40 terminator sequence.

3. The plasmid pSV2-hTPO-EAL-dhfr of about 7.8kb suitable for an expression of a secreting protein part of human thyroid peroxidase retaining activity of antigen with a CHO cell.

4. A CHO cell transformed with the plasmid according to Claim 1.

5. A CHO cell transformed with the plasmid according to Claim 3.

6. A process for preparation of a plasmid suitable for an expression of a secreting protein part of human thyroid peroxidase retaining activity of antigen with a CHO cell, which comprises the steps of:
   (a) cleaving plasmid phTPO-1 with restriction enzyme AccI to obtain a DNA fragment of about 5.7kb, which comprises a sequence coding for part of human thyroid peroxidase region retaining activity of antigen and being substantially free of a sequence coding for a membrane binding region;
   (b) ligating the DNA fragment obtained by said step(a) with a synthetic linker to obtain a DNA fragment ligating linker;
   (c) cleaving the DNA fragment ligating linker obtained by said step(b) with restriction enzyme EcoRI to obtain a DNA fragment of about 2.8kb, which comprises a sequence coding for part of human thyroid peroxidase region, being substantially free of a DNA sequence coding for a membrane binding region;

(d) reacting the DNA fragment obtained by said step(c) with plasmid pUC19 in the presence of T4 DNA ligase to obtain reactant solution, said plasmid pUC19 having been digested with restriction enzyme EcoRI;
(e) transforming E. coli with the reactant solution obtained by said step(d) to obtain an transformant;
(f) recovering plasmid phTPO-EAL from the transformant obtained by said step(e);
(g) cleaving said plasmid phTPO-EAL obtained by said step(f) with restriction enzyme EcoRI to obtain a DNA fragment of about 2.8kb, which comprises a DNA sequence coding for part of human thyroid peroxidase region retaining activity of antigen, being substantially free of a DNA sequence coding for a membrane binding region;
(h) cleaving another plasmid pSV2-dhfr with restriction enzymes HindIII and BglII to obtain a DNA fragment of about 4kb, which comprises sequence of SV40 promoter region, sequence of terminator region and sequence of replication origin in E. coli;
(i) respectively reacting said each DNA fragment obtained by said step(g) and by said step(h) with the Klenow fragment of E. coli in the presence of DNA polymerase I to produce blunt ends on each of the DNA fragments;
(j) reacting two kinds of DNA fragments obtained by said step(i) with T4 DNA ligase to obtain reactant solution;
(k) transforming E. coli with the reactant solution obtained by said step(j) to obtain an ampicillin resistant transformant; and
(l) recovering plasmid pSV2-hTPO-EAL from the transformant obtained by said step(k).
(m) cleaving another plasmid pSV2-dhfr with restriction enzymes PvuII and BglII to obtain a DNA fragment of about 1.1kb, which comprises sequence coding for SV40 promoter region and sequence coding for dihydrofolate reductase region;
(n) cleaving plasmid pSV2-hTPO-EAL obtained by said step(1) with restriction enzyme EcoRI to produce a DNA fragment;
(o) respectively reacting said each DNA fragment obtained by said step(m) and said step(n) with the Klenow fragment of E. coli in the presence of DNA polymerase I to produce blunt ends on each of said DNA fragments;
(p) reacting said DNA fragments obtained by said step(o) with T4 DNA ligase to produce reactant solution;
(q) transforming E. coli with the reactant solution obtained by said step(p) to produce an ampicillin resistant transformant; and
(r) recovering plasmid pSV2-hTPO-EAL-dhfr of about 7.8kb from said transformant obtained by said step(q).

**7.** The process according to Claim 6, wherein said synthetic linker has the nucleotide sequence of

```
5'-AGACTAGTGAATTC-3'
3'    TGATCACTTAAG-5'.
```

**8.** A DNA fragment comprising a DNA sequence coding for part of human thyroid peroxidase region retaining activity of antigen and being substantially free of a DNA sequence coding for a membrane binding region, at one end of said sequence being formed an AccI cutting site.

**9.** A DNA fragment comprising a DNA sequence coding for part of human thyroid peroxidase region retaining activity of antigen and being substantially free of a sequence coding for a membrane binding region;

at one end of said sequence being formed an AccI cutting site and at said AccI cutting site a synthetic linker being ligated;

one of said synthetic linker being capable of ligating AccI cutting site, the other end of said synthetic linker being recognizing site of EcoRI; and

said synthetic linker containing a DNA sequence coding for a stop codon;

**10.** A DNA fragment comprising a DNA coding for part of human thyroid peroxidase region retaining activity of antigen and being substantially free of a DNA sequence coding for a membrane binding region;

at one end of said sequence being formed an AccI cutting site and at said AccI cutting site a synthetic linker being ligated; and

said synthetic linker having the sequence of

5'-AGACTAGTGAATTC-3'
3'    TGATCACTTAAG-5'.

# FIG. 1

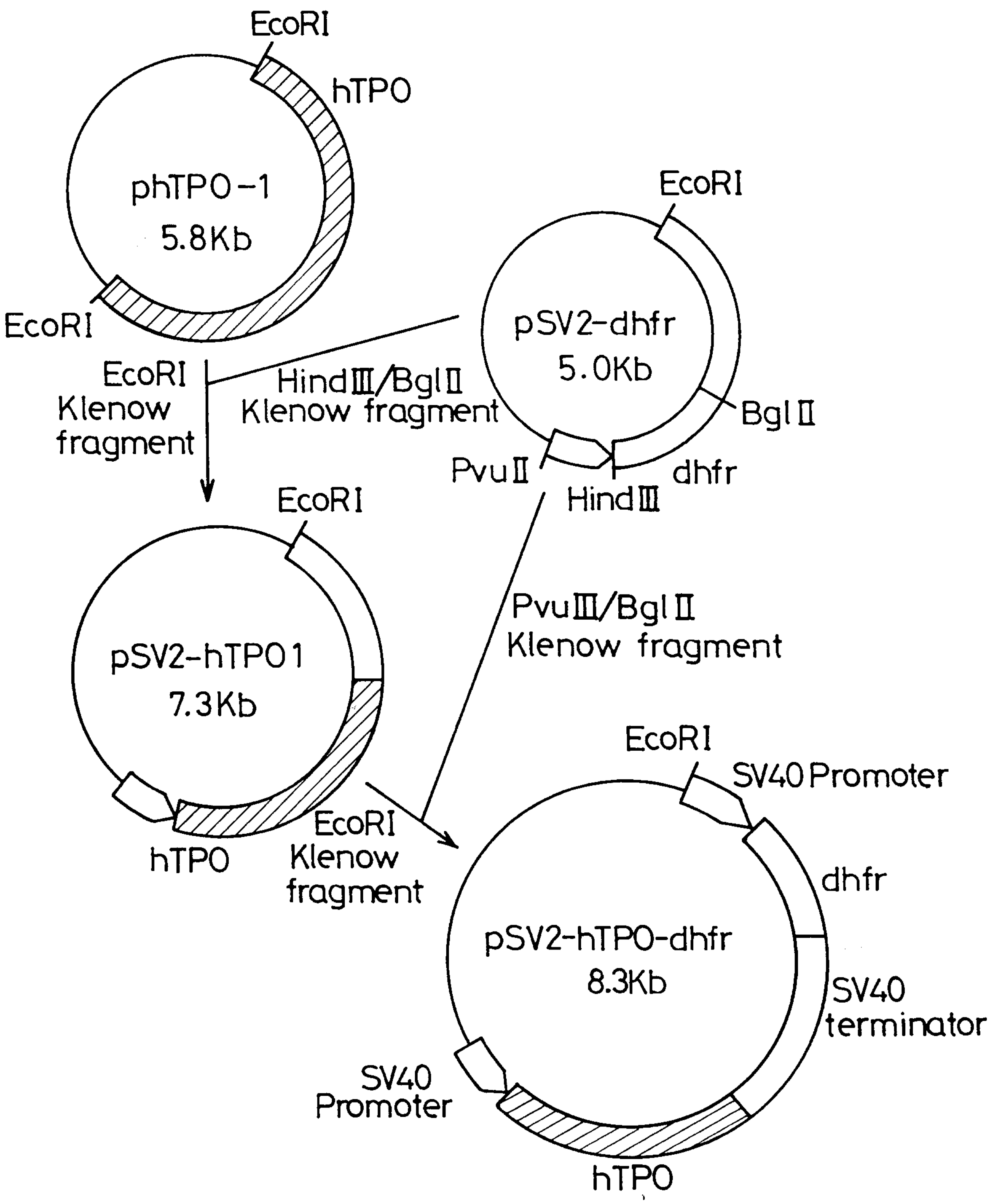
EcoRI
hTPO
phTPO-1
5.8Kb
EcoRI
EcoRI
Klenow
fragment
pSV2-dhfr
5.0Kb
EcoRI
HindIII/BglII
Klenow fragment
BglII
PvuII
HindIII
dhfr
EcoRI
PvuIII/BglII
Klenow fragment
pSV2-hTPO1
7.3Kb
hTPO
EcoRI
Klenow
fragment
EcoRI
SV40 Promoter
dhfr
pSV2-hTPO-dhfr
8.3Kb
SV40
terminator
SV40
Promoter
hTPO

FIG. 2

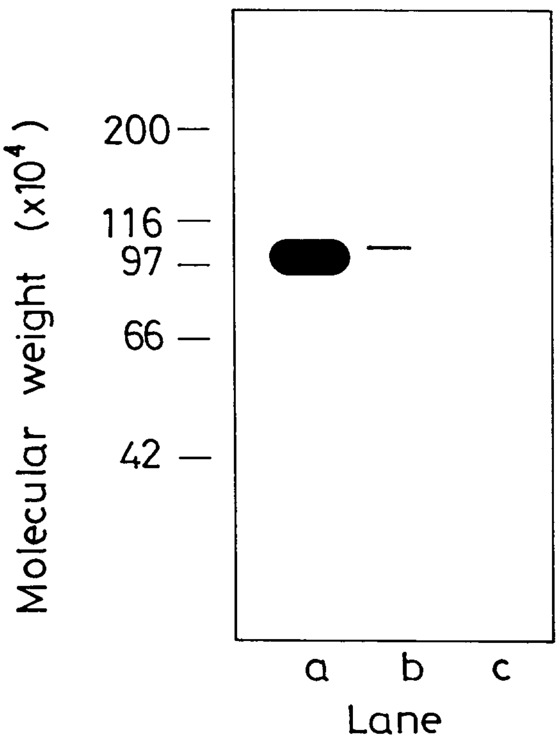


Lane

a : hTPO isolated from human thyroid tissue

b : Product of transformed CHO cells

c : Product of non-transformed CHO cells

FIG. 3

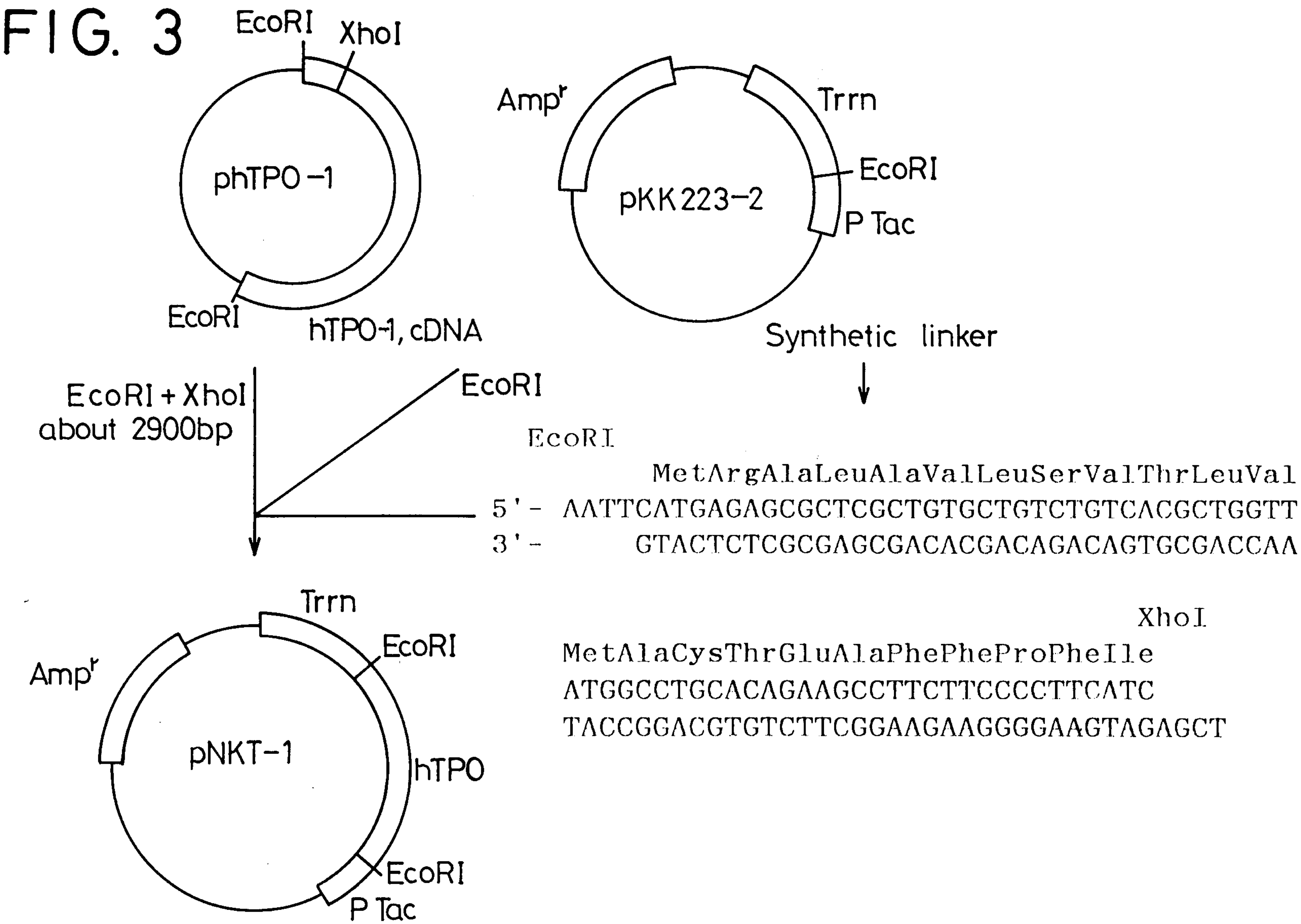
EcoRI
XhoI
phTPO-1
EcoRI
hTPO-1, cDNA
Amp$^r$
pKK223-2
Trrn
EcoRI
P Tac
Synthetic linker
EcoRI + XhoI
about 2900bp
EcoRI
EcoRI
MetArgAlaLeuAlaValLeuSerValThrLeuVal
5'- AATTCATGAGAGCGCTCGCTGTGCTGTCTGTCACGCTGGTT
3'- GTACTCTCGCGAGCGACACGACAGACAGTGCGACCAA
XhoI
MetAlaCysThrGluAlaPhePheProPheIle
ATGGCCTGCACAGAAGCCTTCTTCCCCTTCATC
TACCGGACGTGTCTTCGGAAGAAGGGGAAGTAGAGCT
Trrn
EcoRI
Amp$^r$
pNKT-1
hTPO
EcoRI
P Tac

FIG. 4

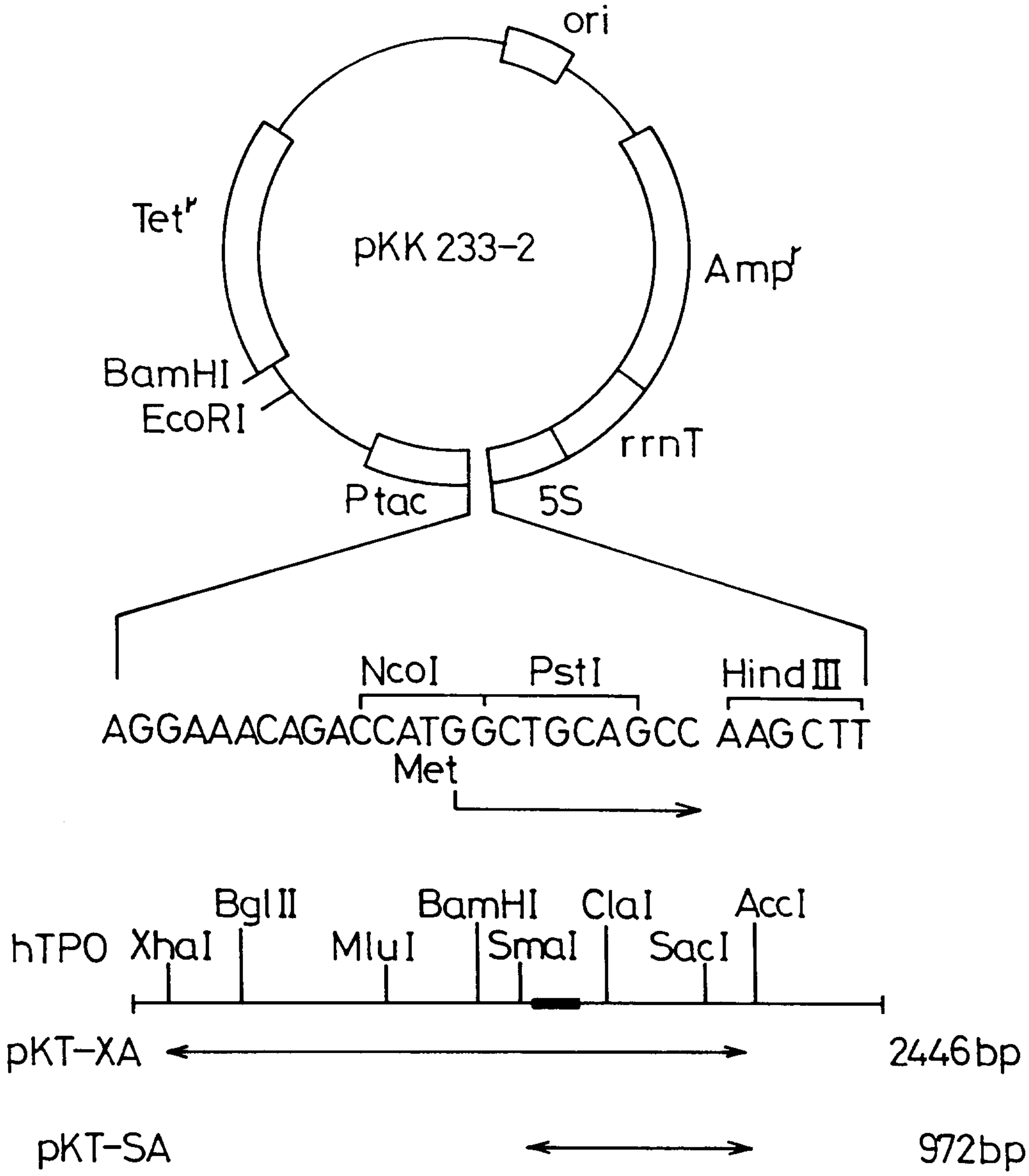
ori
Tet$^r$
pKK 233-2
Amp$^r$
BamHI
EcoRI
rrnT
Ptac
5S
NcoI
PstI
HindIII
AGGAAACAGACCATGGCTGCAGCC AAGCTT
Met
BglII
BamHI
ClaI
AccI
hTPO
XhaI
MluI
SmaI
SacI
pKT-XA
2446bp
pKT-SA
972bp

FIG. 5

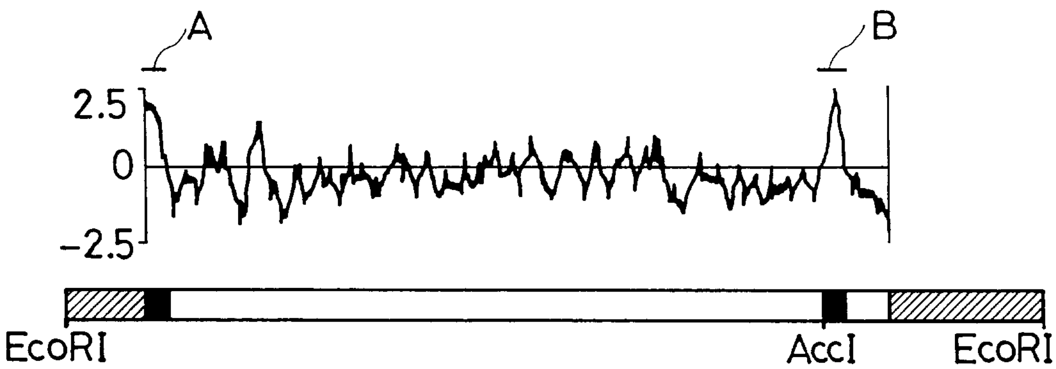


A: Signal sequence
B: Membrane bound region

FIG. 6

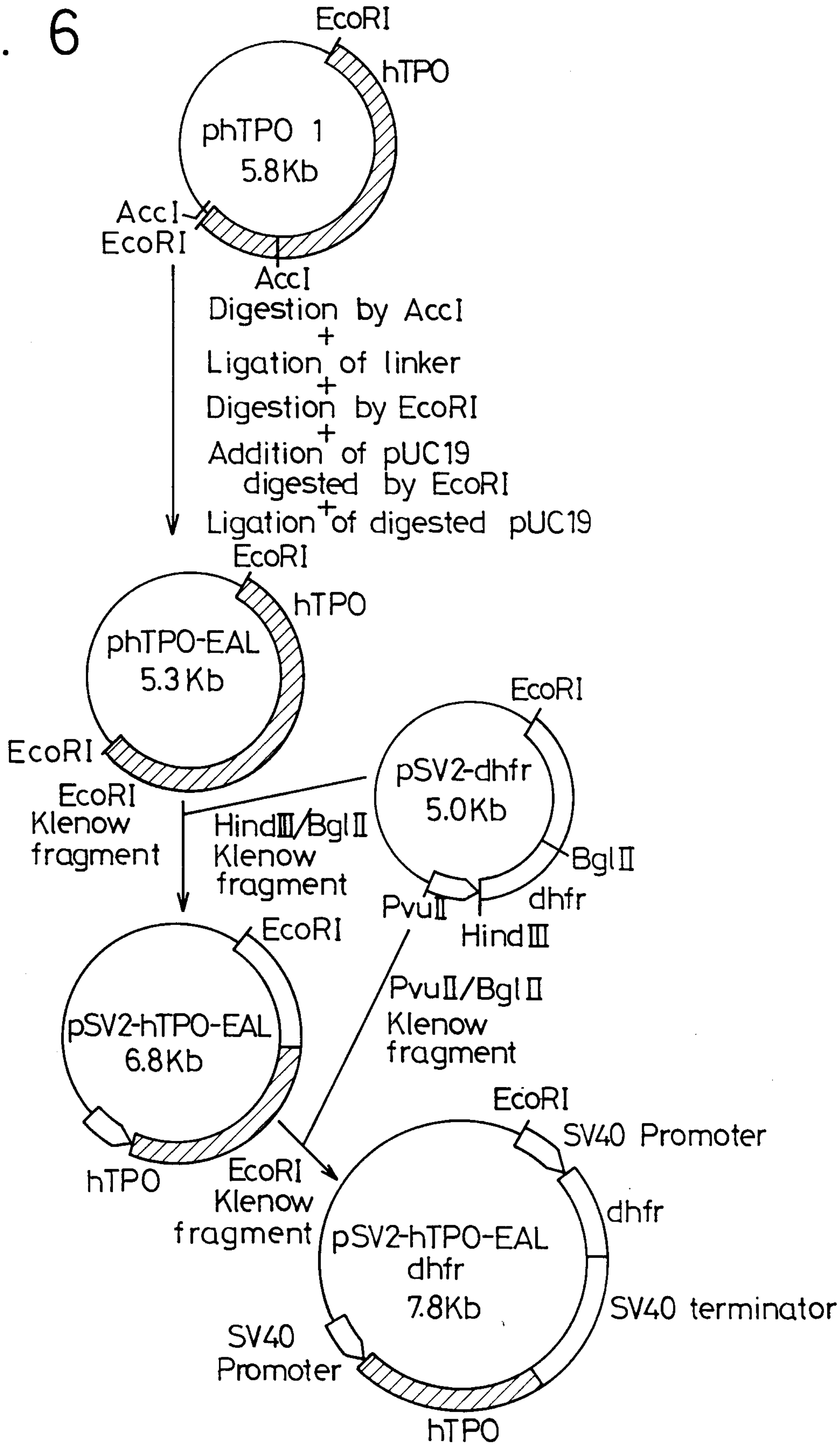
EcoRI
hTPO
phTPO 1
5.8Kb
AccI
EcoRI
AccI
Digestion by AccI
+
Ligation of linker
+
Digestion by EcoRI
+
Addition of pUC19
digested by EcoRI
+
Ligation of digested pUC19
EcoRI
hTPO
phTPO-EAL
5.3Kb
EcoRI
EcoRI
pSV2-dhfr
5.0Kb
BglII
dhfr
PvuII
HindIII
EcoRI
Klenow
fragment
HindIII/BglII
Klenow
fragment
EcoRI
pSV2-hTPO-EAL
6.8Kb
hTPO
PvuII/BglII
Klenow
fragment
EcoRI
Klenow
fragment
EcoRI
SV40 Promoter
dhfr
pSV2-hTPO-EAL
dhfr
7.8Kb
SV40 terminator
SV40
Promoter
hTPO

FIG. 7

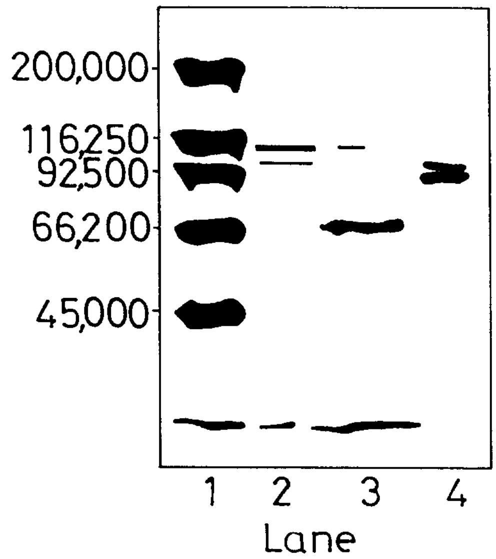


Lane 1 : Molecular weight markers
2 : Membrane bound type hTPO
3 : Secretion type hTPO
4 : Membrane bound type hTPO digested by trypsin

FIG. 8

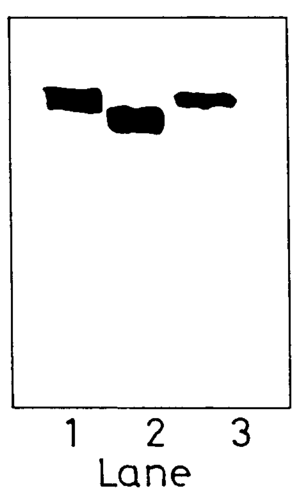


Lane 1 : Membrane bound type hTPO
2 : Membrane bound type hTPO digested by trypsin
3 : Secretion type hTPO

FIG. 9

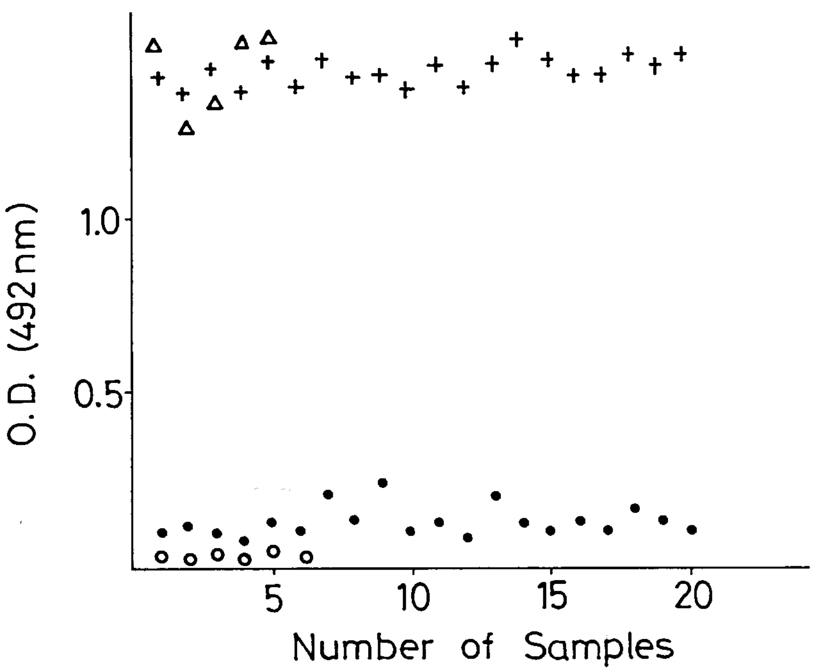


• : Normal serum (Membrane bound type hTPO)

+ : Patient's serum ( ditto )

o : Normal serum (Secretion type hTPO )

Δ : Patient's serum ( ditto )

European Patent Office

# EUROPEAN SEARCH REPORT

Application Number
EP 94 11 7445

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| E | WO-A-91 02061 (RAPAPORT) 21 February 1991<br>--- | 1-10 | C12N15/85<br>C12N5/10<br>C12N15/53 |
| P,X | MOLECULAR ENDOCRINOLOGY,<br>vol.4, no.4, May 1990, BALTIMORE, USA<br>pages 786 - 791<br>D. FOTI ET AL. 'Generation of a biologically active, secreted form of human thyroid peroxidase by site-directed mutagenesis'<br>----- | 1-10 | |

TECHNICAL FIELDS SEARCHED (Int.Cl.5)

C12N
C07K

The present search report has been drawn up for all claims

| Place of search | Date of completion of the search | Examiner |
|---|---|---|
| THE HAGUE | 9 March 1995 | Van Putten, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)